# EUROPEAN PATENT APPLICATION

(11) **EP 1 334 722 A1**
(43) Date of publication of application: **13.08.2003**
(21) Application number: 01978879.3
(22) Date of filing: 24.10.2001
(51) Int. Cl.: A61K 31/198, A61K 9/16, A61P 1/16

(54) **GRANULAR DRUG PREPARATIONS CONTAINING BRANCHED AMINO ACIDS AND PROCESS FOR PRODUCING THE SAME**

(30) Priority: 26.10.2000 JP 2000326513
(71) Applicant: Ajinomoto Co., Inc., Tokyo 104-8315 (JP)
(72) Inventor: IDA, Mitsuyasu, c/o AJINOMOTO CO., INC., Kawasaki-shi, Kanagawa 210-8681 (JP); MAKINO, Chisato, c/o AJINOMOTO CO., INC., Kawasaki-shi, Kanagawa 210-8681 (JP); ORITA, Haruo, c/o AJINOMOTO CO., INC., Kawasaki-shi, Kanagawa 210-8681 (JP); OZAKI, Masanao, c/o AJINOMOTO CO., INC., Kawasaki-shi, Kanagawa 210-8681 (JP)
(74) Representative: Nicholls, Kathryn Margaret
(86) International application number: JP0109332
(87) International publication number: WO02034255

(57) **Abstract**

The present invention provides a granular drug preparation containing isoleucine, leucine and valine as active ingredients, which includes particles of the three kinds of amino acids of isoleucine, leucine and valine as a starting material of granulation, wherein the isoleucine particles and the leucine particles have a particle size of 20-700 µm, and a production method thereof. According to the present invention, bitterness and bad taste characteristic of amino acid of a granular drug preparation containing isoleucine, leucine and valine as active ingredients, which is an effective therapeutic drug of liver diseases, can be effectively suppressed.

## Description

### Technical Field

The present invention relates to a granular drug preparation containing three kinds of branched chain amino acids of isoleucine, leucine and valine as active components, and a production method thereof.

### Background Art

A granular drug preparation containing three kinds of branched chain amino acids of isoleucine, leucine and valine as active components is a therapeutic drug effective for liver diseases. The particle size of a powder to be the starting material of a drug preparation is desired to be small for the retention of content uniformity, and is generally not more than 10 µm. However, a granular preparation containing these three kinds of amino acids is defective in that it is bitter, has bad taste and is extremely difficult to take, when the particle size of the branched chain amino acids is small.

By adding sucrose, aspartame, saccharin and the like commonly used as sweeteners to granules, or by forming a corrective coating layer thereof, bitterness and bad taste can be reduced, but it is not sufficient.

### Disclosure of the Invention

The present invention aims at provision of a granular drug preparation containing three kinds of branched chain amino acids of isoleucine, leucine and valine, wherein bitterness and bad taste characteristic of the three kinds of branched chain amino acids of isoleucine, leucine and valine, which are effective therapeutic drugs of liver diseases, have been reduced to the extent that it can be taken without using a sweetener or forming a coating layer such as corrective coating layer.

The present invention capable of solving the above-mentioned problem is based on granulation while adjusting isoleucine particles and leucine particles used for the production of a granular preparation containing particles of three kinds of amino acids of isoleucine, leucine and valine to have a particle size greater than that of particles used for granulation to give conventional preparations, and encompasses each of the following inventions.
(1) A production method of a granular drug preparation containing three kinds of branched chain amino acids of isoleucine, leucine and valine as active components, which comprises granulating a starting material for granulation, containing particles of the three kinds of branched chain amino acids of isoleucine, leucine and valine, wherein the isoleucine particles and the leucine particles have an adjusted particle size of 20-700 µm.
(2) The production method of a granular drug preparation containing three kinds of branched chain amino acids of isoleucine, leucine and valine as active components according to the above-mentioned (1), wherein the aforementioned isoleucine particles and the leucine particles have a particle size of 50-500 µm.
(3) The production method of a granular drug preparation containing three kinds of branched chain amino acids of isoleucine, leucine and valine as active components according to the above-mentioned (1) or (2), wherein the aforementioned isoleucine, leucine and valine are contained in a weight ratio of isoleucine/leucine/valine=1/1.9-2.2/1.1-1.3.
(4) A granular drug preparation containing three kinds of branched chain amino acids of isoleucine, leucine and valine as active components, which comprises particles of the three kinds of amino acids of isoleucine, leucine and valine, wherein the isoleucine particles and the leucine particles have a particle size of 20-700 µm.
(5) The granular drug preparation containing three kinds of branched chain amino acids of isoleucine, leucine and valine as active components according to (4), wherein the aforementioned isoleucine particles and the leucine particles have a particle size of 50-500 µm.
(6) The granular drug preparation containing three kinds of branched chain amino acids of isoleucine, leucine and valine as active components according to the above-mentioned (4) or (5), wherein the aforementioned isoleucine, leucine and valine are contained in a weight ratio of isoleucine/leucine/valine=1/1.9-2.2/1.1-1.3.

The granular drug preparation of the present invention is produced by granulating the particles of three kinds of branched chain amino acids of isoleucine, leucine and valine.

The granular drug preparation of the present invention refers to the granules and powders described in the Japanese Pharmacopoeia or the European Pharmacopoeia.

In the granular drug preparation of the present invention, isoleucine, which is one of the active components, is, but not limited to, a particle having a particle size of not more than 1 mm, which is generally produced by a fermentation method and satisfies any of the standards of the Japanese Pharmacopoeia, the European Pharmacopoeia and the United States Pharmacopoeia. It is adjusted to a particle size of 20-700 µm, preferably 50-500 µm, and used.

Leucine is, but not limited to, a particle having a particle size of not more than 1 mm, which is generally produced by a fermentation method or an extraction method and satisfies any of the standards of the Japanese Pharmacopoeia, the European Pharmacopoeia and the United States Pharmacopoeia. It is adjusted to a particle size of 20-700 µm, preferably 50-500 µm, and used.

Valine is, but not limited to, a particle having a particle size of not more than 1 mm, which is generally produced by a fermentation method or a synthetic method and satisfies any of the standard of the Japanese Pharmacopoeia, the European Pharmacopoeia and the United States
Pharmacopoeia. As long as valine satisfies any of the standard of the Pharmacopoeia, its particle size is not particularly limited.

The method for adjusting the particle size of the particles of the three kinds of branched chain amino acids of isoleucine, leucine and valine used for granulation is not particularly limited, and a conventional pulverization method is employed. Pulverizers usable for pulverization includes impact type (high speed rotation type) pulverizers such as a hammer mill and the like, tumbler type (medium using type) pulverizers such as a ball mill and the like and fluid type (stream type) pulverizers such as a jet mill and the like.

The mixing ratio of the three kinds of branched chain amino acids of isoleucine, leucine and valine in the granular preparation of the present invention in a weight ratio is isoleucine/leucine/valine=1/1.9-2.2/1.1-1.3.

For production of the granular preparation of the present invention, a binder can be used. As the binder, cellulose derivatives such as methyl cellulose, hydroxypropyl cellulose, hydroxypropyl methylcellulose, hydroxypropyl methylcellulose phthalate and the like, starches such as corn starch, wheat starch and the like, synthetic polymers such as polyvinylpyrrolidone, acrylic polymer and the like, natural polymers such as acacia, gelatin and the like, and the like can be used without any particularly limitation as long as it can be used for pharmaceutical agents. In addition, its amount of use may be any as long as it is within the range that affords conventional granulation.

The particle size of a powder to be the starting material of a pharmaceutical preparation is preferably small to retain content uniformity, which is generally not more than 10 µm.

However, due to the high content and high ratio per dose of the particles of the three kinds of amino acids consisting of isoleucine, leucine and valine, which are the active components, the granular preparation of the present invention is advantageous in that the content ratio of the three kinds of amino acid components in the granules can be maintained to be constant even when the three kinds of amino acid components have a greater particle size.

In the granular drug preparation of the present invention, the granule containing the particles of the aforementioned three kinds of amino acids can be prepared using any equipment of a high-speed agitation granulator, a fluidized bed granulator, a planetary mixer, a dry pressing granulator, a pulverizing granulator, an extrusion granulator, a rotating granulator, a spray dry granulator and a coating granulator, with preference given to a high-speed agitation granulator and an extrusion granulator.

The extrusion granulation method produces granules by extruding a plasticized powder from a screen having many holes, and employs a forward extrusion granulator, a disk pelleter granulator, a ring die granulator, a basket granulator, an oscillation granulator, a cylinder granulator and the like.

The high-speed agitation granulation method comprises casting or spraying water or a binder solution to a powder, and shearing, rotating and consolidating the mixture by the rotation of agitating blade to give granules, for which a vertical and horizontal agitation granulators are used.

The fluidized bed granulation method comprises spraying water or a binder solution while fluidizing the powder to allow agglutination, for which a fluidized bed granulator, an agitation fluidized bed granulator, a rotating fluidized bed granulator and a rotating agitation fluidized bed granulator are used.

The dry pressing granulation method comprises compression forming a powder without using water or a binder solution, for which a roll press, a briquetting machine, a single punch tablet press and a rotary tablet press are used.

The rotating granulation method comprises rotating a powder for granulation, for which a drum granulator, a pan granulator, oscillating granulator and a rotating disc granulator are used.

It is needless to say that the bitterness of the granular preparation of the present invention, which is produced by granulation in the above-mentioned granulators, can be further reduced by adding some amount of a sweetening component or applying some amount of a coating during granulation.

### Best Mode of Embodying the Invention

While the present invention is now explained in more detail by referring to Examples, the present invention is not limited by the following Examples.

The kinds and particle size of the branched chain amino acids used in each Example are shown in the following Table 1, wherein the particle size was measured by the following method.

Using a laser diffraction/scattering particle size distribution measurement instrument (manufactured by Horiba, Ltd., LA-910), 2-propanol (200 ml) is placed in a circulation tank and circulated for 5 min with stirring and ultrasonic irradiation, which is followed by blank measurement (ultrasonic OFF during measurement). Subsequently, 2-propanol (200 ml) is placed in the circulation tank and amino acid specimen for measurement is cast such that the transmittance becomes about 85%. The mixture is circulated with stirring and ultrasonic irradiation for 4 min, and at 5 min after the ultrasonic irradiation is ceased, the particle size is measured. For the mean particle size, the median size based on volume is used.

**Table 1**

| BCAA | Volume-based median size |
|---|---|
| L-leucine | 411 µm |
| | 267 µm |
| | 59 µm |
| | 23 µm |
| L-isoleucine | 51 µm |
| | 28 µm |
| L-valine | 179 µm |
| | 45 µm |
| | 22 µm |

### Example 1

| (Formulation 1) | particle size | amount added |
|---|---|---|
| L-leucine | 411 µm | 76.16 g |
| L-isoleucine | 51 µm | 38.00 g |
| L-valine | 179 µm | 45.76 g |
| hydroxypropyl cellulose (type M) | | 4.80 g |

### Production method

Using branched chain amino acid having the particle size shown in the above-mentioned Formulation 1, granulation particles were obtained. That is, a given amount of each component shown in Formulation 1 was weighed, uniformly mixed in an agitation granulator (manufactured by Fukae Powtec Co., Ltd., high-speed mixer), and distilled water was added for granulation. The granulated product was dried at 60°C for 2 hrs using a column dryer to give granules. After passing a 1400 µm sieve, an oversized product was force sieved using a 1400 µm sieve, thoroughly mixed with an undersized product to give the granular preparation of the present invention.

### Example 2

| (Formulation 2) | particle size | amount added |
|---|---|---|
| L-leucine | 411 µm | 76.16 g |
| L-isoleucine | 51 µm | 38.00 g |
| L-valine | 22 µm | 45.76 g |
| hydroxypropyl cellulose (type M) | | 4.80 g |

### Production method

In the same manner as in Example 1 except that branched chain amino acid having a particle size shown in Formulation 2 was used instead of the branched chain amino acid having a particle size used in Example 1, a granular preparation was obtained.

### Example 3

| (Formulation 3) | particle size | amount added |
|---|---|---|
| L-leucine | 267 µm | 76.16 g |
| L-isoleucine | 51 µm | 38.00 g |
| L-valine | 22 µm | 45.76 g |
| hydroxypropyl cellulose (type M) | | 4.80 g |

### Production method

In the same manner as in Example 1 except that branched chain amino acid having a particle size shown in Formulation 3 was used instead of the branched chain amino acid having a particle size used in Example 1, a granular preparation was obtained.

### Example 4

| (Formulation 4) | particle size | amount added |
|---|---|---|
| L-leucine | 59 µm | 76.16 g |
| L-isoleucine | 51 µm | 38.00 g |
| L-valine | 22 µm | 45.76 g |
| hydroxypropyl cellulose (type M) | | 4.80 g |

### Production method

In the same manner as in Example 1 except that branched chain amino acid having a particle size shown in Formulation 4 was used instead of the branched chain amino acid having a particle size used in Example 1, a granular preparation was obtained.

### Example 5

| (Formulation 5) | particle size | amount added |
|---|---|---|
| L-leucine | 23 µm | 76.16 g |
| L-isoleucine | 51 µm | 38.00 g |
| L-valine | 22 µm | 45.76 g |
| hydroxypropyl cellulose (type M) | | 4.80 g |

### Production method

In the same manner as in Example 1 except that branched chain amino acid having a particle size shown in Formulation 5 was used instead of the branched chain amino acid having a particle size used in Example 1, a granular preparation was obtained.

### Example 6

| (Formulation 6) | particle size | amount added |
|---|---|---|
| L-leucine | 23 µm | 76.16 g |
| L-isoleucine | 28 µm | 38.00 g |
| L-valine | 22 µm | 45.76 g |
| hydroxypropyl cellulose (type M) | | 4.80 g |

### Production method

In the same manner as in Example 1 except that branched chain amino acid having a particle size shown in Formulation 6 was used instead of the branched chain amino acid having a particle size used in Example 1, a granular preparation was obtained.

### Experimental Example

The granular preparations (4 g as branched chain amino acid) obtained in Example 1 to Example 6 were given as they were in granules to 6 adult male panelists (a - f) while securing the blindness, and sensory evaluation was made. As medication method, the granules were taken with water (50 ml), the bitterness immediately after medication and aftertaste (bitterness after 1 min of medication) were evaluated according to the following criteria.
⊙ bitterness not at all bothering, ○ bitterness not bothering very much, Δ bitterness somewhat bothering, × bothering bitterness

The results are shown in Table 2.

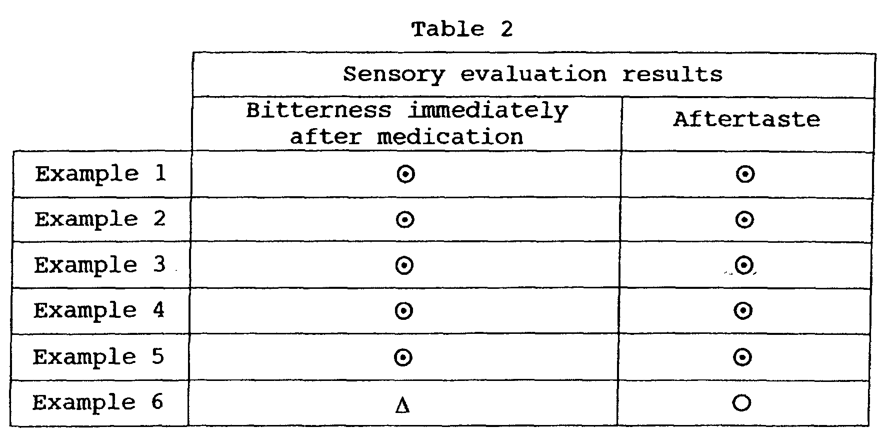

As is clear from Table 2, the combination of branched chain amino acids having a controlled particle size enabled preparation of a granular preparation with suppressed expression of bitterness, even without addition of a sweetener.

### Comparative Example

A jet mill pulverized product of branched chain amino acids (340.4 g, L-leucine:L-isoleucine:L-valine=2:1:1.2 (weight ratio), particle size 4 µm) and 5.52 g of hydroxypropyl cellulose (type H) were mixed in a "Shinagawa" kneader (Dalton multi-purpose mixer DM-03 type) for 3 min and distilled water (117.5 g) was added, which was followed by kneading for 5 min. The obtained kneaded product was prepared into columnar granules with a "Dome gran" extrusion granulator set with a 0.6 mm screen, and the granules were dried in a column dryer at 60°C overnight to give granule A.

By a similar operation using branched chain amino acid having a particle size of 55 µm, granule B was obtained.

For sensory evaluation, 1 g each of granule A and granule B obtained above was evaluated by the aforementioned 6 test subjects in the order of A→B (3 subjects) and B→A (3 subjects) according to the following evaluation criteria. The results are shown in Table 3.
bitterness: ⊙ not at all bothering, ○ somewhat bothering, Δ considerably bothering, × very strong
foul flavor: ⊙ not at all bothering, ○ somewhat bothering, Δ considerably bothering, × very strong

**Table 3**

| Test subject | a | b | c | d | e | f | overall |
|---|---|---|---|---|---|---|---|
| Evaluation order | A→B | A→B | A→B | B→A | B→A | B→A | |
| Bitterness A | × | × | Δ | × | × | × | x |
| Bitterness B | Δ | ○ | Δ | Δ | ○ | Δ | Δ |
| Foul flavor A | ○ | Δ | Δ | ○ | Δ | Δ | Δ |
| Foul flavor B | ○ | ○ | ○ | ○ | Δ | Δ | ○ |

As is evident from Table 3, the granules made from finely pulverized branched chain amino acid as a starting material showed both strong bitterness and foul flavor, though subject to certain dispersion depending on the individuals who made the evaluation, which led to the confirmation of a great influence of the particle size of the starting material, branched chain amino acid, in the improvement of taste and flavor.

### Industrial Applicability

As mentioned above, in a granular drug preparation containing three kinds of branched chain amino acids of isoleucine, leucine and valine, effects that the bitterness specific to the amino acid component can be reduced and the preparation becomes easy to take can be achieved by making the particle sizes of isoleucine and leucine greater as in the present invention.

## Claims

1. A production method of a granular drug preparation containing three kinds of branched chain amino acids of isoleucine, leucine and valine as active components, which comprises granulating a starting material for granulation, containing particles of the three kinds of branched chain amino acids of isoleucine, leucine and valine, wherein the isoleucine particles and the leucine particles have an adjusted particle size of 20-700 µm.

2. The production method of a granular drug preparation containing three kinds of branched chain amino acids of isoleucine, leucine and valine as active components according to claim 1, wherein said isoleucine particles and the leucine particles have a particle size of 50-500 µm.

3. The production method of a granular drug preparation containing three kinds of branched chain amino acids of isoleucine, leucine and valine as active components according to claim 1 or 2, wherein said isoleucine, leucine and valine are contained in a weight ratio of isoleucine/leucine/valine=1/1.9-2.2/1.1-1.3.

4. A granular drug preparation containing three kinds of branched chain amino acids of isoleucine, leucine and valine as active components, which comprises particles of the three kinds of amino acids of isoleucine, leucine and valine, wherein the isoleucine particles and the leucine particles have a particle size of 20-700 µm.

5. The granular drug preparation containing three kinds of branched chain amino acids of isoleucine, leucine and valine as active components according to claim 4, wherein said isoleucine particles and the leucine particles have a particle size of 50-500 µm.

6. The granular drug preparation containing three kinds of branched chain amino acids of isoleucine, leucine and valine as active components according to claim 4 or 5, wherein said isoleucine, leucine and valine are contained in a weight ratio of isoleucine/leucine/valine=1/1.9-2.2/1.1-1.3.
